# NEUE EUROPÄISCHE PATENTSCHRIFT

(11) **EP 1 133 555 B2**
(45) Veröffentlichungstag und Bekanntmachung des Hinweises auf die Entscheidung über den Einspruch: **04.09.2013**
(45) Hinweis auf die Patenterteilung: 23.11.2005
(21) Anmeldenummer: 99926183.7
(22) Anmeldetag: 14.06.1999
(51) Int. Cl.: C12N 9/64, A61K 38/36

(54) **PHARMAZEUTISCHES FAKTOR VII-PRÄPARAT**
PHARMACEUTICAL FACTOR VII PREPARATION
PREPARATION PHARMACEUTIQUE A BASE D'UN FACTEUR VII

(30) Priorität: 17.06.1998 AT 104398
(43) Veröffentlichungstag der Anmeldung: 19.09.2001
(73) Patentinhaber: Baxter Aktiengesellschaft, 1221 Wien (AT)
(72) Erfinder: MATTHIESSEN, Peter, 1020 Wien (AT); TURECEK, Peter, 3400 Klosterneuburg (AT); SCHWARZ, Hans-Peter, 1180 Wien (AT)
(74) Vertreter: Alge, Daniel
(86) Internationale Anmeldenummer: PCT/AT1999/000154
(87) Internationale Veröffentlichungsnummer: WO 1999/066031

(56) Entgegenhaltungen:
- EP-A- 0 129 634
- EP-A- 0 770 625
- WO-A-94/22905
- DE-A- 19 531 637
- BAJAJ S P ET AL: "Isolation and charcterization of human factor VII" JOURNAL OF BIOLOGICAL CHEMISTRY., Bd. 256, Nr. 1, 1981, Seiten 253-259, XP002118780 MD US

## Beschreibung

Die Erfindung betrifft ein Verfahren zur Reinigung von Blutgerinnungsfaktor VII.

Die Blutgerinnung wird durch eine Reihe von aufeinanderfolgenden Reaktionen verschiedener Blutgerinnungsfaktoren ausgelöst. Durch einen Mangel an Blutgerinnungsfaktoren wird die Bildung von Fibrin aus Fibrinogen und damit der Wundverschluß verhindert; die Folge ist ein erhöhtes Blutungsrisiko bzw. Blutungen. Ein solcher Fall liegt bei einem Mangel von Vitamin K-abhängigen Blutgerinnungsfaktoren, wie den Faktoren II, VII, IX und X vor, der vor allem durch die Beeinträchtigung der Leberfunktion, aber auch aufgrund eines vererbten Blutgerinnungsfaktormangel hervorgerufen sein kann. Zur Substitutionsbehandlung werden die entsprechenden Blutgerinnungsfaktoren eingesetzt. Die Behandlung mit diesen Präparaten führt in den meisten Fällen zu einer raschen Blutstillung.

Der Faktor VII kann aus biologischem Material, wie Blut, Plasma oder Zellkulturen gewonnen werden und wird im Fall von Blut oder Plasma als Ausgangsmaterial zumeist gemeinsam mit mindestens einem der strukturell ähnlichen Faktoren II, IX oder X in gereinigter Form erhalten. Ein Prothrombinkomplex-Präparat auf Basis der Faktoren II, VII, IX und X bzw. eine Plasmafraktion enthaltend den Prothrombinkomplex, kann ebenfalls als Ausgangsmaterial zur Herstellung eines weiter gereinigten Faktor VII-Präparates herangezogen werden.

Zur Behandlung von Patienten, bei denen ein Mangel an Faktor VIII auftritt und welche einen gegen den Faktor VIII gerichteten Hemmstoff (Inhibitor) entwickelt haben wird oftmals ein Faktor VIIa-Präparat vorgeschlagen. Hochgereinigte Faktor VIIa-Präparate werden z.B. in der EP 0 082 182 und von Hedner et al. (Haemostasis 19, 335-343 (1989)) beschrieben.

Der Faktor VII ist relativ leicht aktivierbar zum Faktor VIIa. Es wurde beispielsweise gefunden, daß Faktor VII-Zymogen durch eine Reihe von physiologischen Enzymen, wie Faktor IXa und Faktor Xa rasch aktiviert wird (Wildgoose et al., Blood, Vol. 73, No. 7, 1989, pp 1888-1895).

In der EP 0 770 625 ist beschrieben, daß mit zunehmenden Aufwand eines Reinigungsvorganges die Aktivierung des Faktor VII eintritt. Zur Vorbeugung des Risikos zur Faktor VIIa-Bildung wird dementsprechend der Zusatz von Inhibitoren der Blutgerinnung, beispielsweise Antithrombin III/Heparin oder reversible Inhibitoren, wie Benzamidin, während einer affinitätschromatographischen Reinigung vorgeschlagen.

Gleichermaßen wird das Faktor VII-Molekül während dessen Isolierung durch die Verwendung von Benzamidin während des gesamten Reinigungsverfahrens vor Proteolyse geschützt, wie von Radcliffe R et al., Journal Biological Chemistry 250, 1975, pp 388-395, beschrieben.

Das Problem der Aktivierung des Faktor VII vor allem in Gegenwart von Oberflächen mit positiver Ladung, z.B. bei Kontakt mit einem Anionenaustauschermaterial, wird von Pedersen et al. (Biochemistry, 28, 1989, 9331-9336) beschrieben. Dabei wurde gefunden, daß rekombinanter Faktor VII in Gegenwart von Benzamidin zu einem homogenen Protein gereinigt werden konnte. In Abwesenheit des Inhibitors wurde der rekombinante Faktor VII spontan aktiviert. Diese autokatalytische Aktivierung stellt daher auch bei Präparationen ein Problem dar, bei welchen die physiologischen Aktivierungskomponenten nicht einmal mehr in Spuren vorhanden sind.

Inhibitoren der Blutgerinnung sind in einem pharmazeutischen Präparat zur Behandlung von Zuständen, die auf einen Mangel eines Blutgerinnungsfaktors zurückzuführen sind, an sich nicht erwünscht. Zwar werden aus Stabilitätsgründen, nämlich um die Aktivierung der Blutgerinnungsfaktoren in Prothrombinkomplex-Präparaten, zu vermeiden, physiologische Inhibitoren, wie Antithrombin III oder Heparin zugesetzt. Es wäre jedoch wünschenswert, Präparate bereitzustellen, die möglichst ohne den Zusatz derartiger Inhibitoren eine ausreichende Stabilität aufweisen.

Im Sigma Katalog, 1997 wird eine Faktor VII-Präparation beschrieben, die mit 1 mM Benzamidin HCl versetzt ist, einem Inhibitor der Blutgerinnung.

Die DE 195 31 637 A betrifft eine Präparation umfassend sowohl Faktor VII als auch Faktor VIIa. In dieser Schrift wird nicht offenbart, dass die Präparation einen geringen Anteil an Faktor VIIa umfassen soll. Da diese Präparation zwingend einen maßgeblichen Anteil an aktiviertem Faktor VII aufweisen muss, weist dieses Dokument geradezu von der vorliegenden Erfindung weg.

In der WO 94/22905 A wird die Reinigung von Faktor VII mittels Gel-Chromatographie beschrieben, wobei den Elutionspuffern Zinkionen zugesetzt werden, um somit die Aktivierung von Faktor VII zu inhibieren. Ausgehend von diesem Dokument als nächster Stand der Technik ist als Aufgabe der vorliegenden Erfindung anzusehen, ein Verfahren zur Verfügung zu stellen, das zu einem stabilen Faktor VII-Präparat führt, das weniger als 5% Faktor VIIa bezogen auf die Gesamtmenge von Faktor VII aufweist, welches Präparat jedoch frei ist von Inhibitoren der Blutgerinnung sowie auch ein Präparat erhältlich durch dieses Verfahren.

Bajaj et al. (Journal of Biological Chemistry 256 (1) (1981), 253-259) betrifft ein Faktor VII-Präparat umfassend Benzamidin, so dass Faktor VII während der Lagerung nicht weiter aktiviert wird. Es wird weiter beschrieben, dass nach Entfernung von Benzamidin die Präparation eine weitere Aktivierung von einem Faktor VII/VIIa-Verhältnis von 1,5 zu einem Verhältnis von 2,5 erfuhr, was die Gegenwart von etwa 10% aktiviertem Faktor VII entspricht. Damit ist auch gemäß diesem Dokument ein Inhibitor der Blutgerinnung notwendig, um ein lagerstabiles Produkt umfassend Faktor VII zur Verfügung zu stellen.

Die Erfindung stellt sich zur Aufgabe ein pharmazeutisches Faktor VII-Präparat bereitzustellen, mit einem möglichst geringen Anteil an aktiviertem Faktor VII, mit einer ausreichenden Stabilität in Abwesenheit von Inhibitoren der Blutgerinnung. Darüber hinaus soll ein Reinigungsverfahren zur Herstellung von Faktor VII-Präparaten zur Verfügung gestellt werden, welches in effizienter und schonender Weise durchgeführt werden kann, um auf den Einsatz von Inhibitoren, wie Benzamidin, zu verzichten.

Die Aufgabe wird erfindungsgemäß gelöst durch ein Verfahren zur Reinigung von Faktor VII aus einem biologischen Material und Herstellung eines Faktors VII-Präparates durch Adsorption des Faktor VII an einem Anionenaustauscher in einer Säule fraktionierte Elution des Faktor VII mit einer spezifischen amidolytischen Aktivität von mindestens 50 E/mg, wobei die Elution mit einem Puffer ohne Zusatz von Inhibitoren der Blutgerinnung vorgenommen wird und die Flussrate der Elution mindestens 0,15 Säulenvolumina pro Minute beträgt, und Gewinnen des Faktor VII aus dem Eluat, so dass das Faktor VII-Präparat einen Anteil von weniger als 5% Faktor VIIa bezogen auf die Gesamtmenge Faktor VII aufweist.

Als Ausgangsmaterial zur Herstellung des erfindungsgemäßen Faktor VII-Präparates wird üblicherweise ein komplexes biologisches Material eingesetzt. Dazu zählen Blut, Plasma, Plasmafraktionen, Zellkulturen bzw. Zellkulturfraktionen. Es kann aber auch ein Präparat mit pharmazeutischer Qualität als Ausgangsmaterial eingesetzt werden, welches neben dem Faktor VII auch weitere Proteine enthält, beispielsweise ein Prothrombinkomplex-Präparat enthaltend die Faktoren II, VII, IX und X.

Zu Vermeidung des Risikos der Übertragung von humanpathogenen Infektionserregern, darunter die von durch Blut übertragbaren Viren, wie HIV und Hepatitisviren, z.B. HAV, HBV, HCV, HGV, und Parvoviren, aber auch die infektiösen Erreger von BSE und CJD, werden eine Reihe von Maßnahmen vorgenommen. Der Faktor VII kann jeweils vor oder nach der chromatographischen Reinigung einem Verfahren zur Inaktivierung bzw. Abreicherung der Humanpathogene unterzogen werden. Vorzugsweise werden mindestens zwei Maßnahmen vorgenommen, die die Inaktivierung bzw. Abreicherung aufgrund eines unterschiedlichen Mechanismus bewirken. Dazu zählen chemische, chemisch-physikalische und physikalische Methoden. Die Methoden unter Einsatz von viruziden Substanzen werden vorzugsweise vor bzw. während des chromatographischen Reinigungsverfahrens vorgenommen, damit gleichzeitig mit der Reinigung des Faktor VII auch das viruzide Mittel entfernt werden kann.

Zu den effektiven Maßnahmen zur Inaktivierung von Viren zählen beispielsweise die Behandlung mit organischen Lösungsmitteln und/oder Detergenzien (EP 0 131 740, EP 0 050 061, PCT/AT98/00090), die Behandlung mit chaotropen Mitteln (WO 90/15613) Hitzebehandlungsverfahren, vorzugsweise in lyophilisiertem, trockenen oder feuchten Zustand (siehe EP 0 159 311), Kombinationsverfahren (EP 0 519 901) und physikalische Methoden. Letztere bewirken die Inaktivierung von Viren beispielsweise durch Bestrahlung mit Licht, etwa in Gegenwart von Photosensibilisatoren (EP 0 471 794 und WO 97/37686).

Zu den Abreicherungsverfahren der Humanpathogene zählen insbesondere die Filtrationen unter Verwendung von Ultrafiltern, Tiefenfiltern oder Nanofiltern (s. WO 97/40861, AT A 1029/97). Aber auch Fällungsschritte bzw. andere Proteinreinigungs-maßnahmen, wie die der Adsorption, tragen zur Abreicherung von möglicherweise vorhandenen Pathogenen bei.

Das erfindungsgemäße Verfahren zur Reinigung von Faktor VII und Herstellung eines Faktor VII-Präparates umfasst mindestens eine Chromatographiestufe. Dabei wird der Faktor VII adsorbiert und selektiv eluiert, wobei Fraktionen gewonnen werden. Zur weiteren Gewinnung des Faktor VII aus dem Eluat werden diejenigen Fraktionen gewählt, in der die spezifische Aktivität mindestens 50 E/mg Protein, vorzugsweise mindestens 100 E/mg, beträgt, vorzugsweise mindestens die zuvor genannten höher gereinigten Proteine.

Als Elutionspuffer wird vorzugsweise ein Puffer mit einem pH im neutralen Bereich, etwa im Bereich von 5-9, vorzugsweise von 6-7,5, eingesetzt und einer Ionenstärke entsprechend einem Gehalt an NaCl von weniger als 1 M eingesetzt. Wie bereits zuvor beschrieben, werden dem Elutionspuffer keine der genannten Inhibitoren der Blutgerinnung zugesetzt. Eventuell vorhandene physiologische Inhibitoren, die im Ausgangsmaterial vorliegen, werden bereits bei der Adsorption und gegebenenfalls bei der darauffolgenden Reinigung des adsorbierten Faktor VII mit einem Waschpuffer abgetrennt, so dass der Faktor VII jedenfalls ohne Inhibitorgehalt gewonnen wird. Auch hier zeigt sich die außerordentliche Stabilität des hochgereinigten Faktor VII, der dann den weiteren üblichen Aufbereitungsmaßnahmen zur Herstellung eines pharmazeutischen oder diagnostischen Präparates unterzogen werden kann.

Die Chromatographie wird in einer Anionenaustauschsäule durchgeführt. Zur verbesserten Kontrolle der Flussrate bzw. der Kontaktzeit des Faktor VII mit dem Chromatographiematerial wird das Säulenverfahren eingesetzt.

Als Anionenaustauscher kommen im Prinzip alle Anionenaustauscher auf Basis von Kohlenhydraten oder synthetischen Polymeren in Frage, die eine Affinität zu Faktor VII (Prothrombin) aufweisen, wie z.B. DEAE-Sephacel®, DEAE-Sephadex®, DEAE-Sepharose CL6B®, DEAE-Sepharose Fast Flow®, QAE-Sephadex®, Q-Sepharose Fast Flow®, Q-Sepharose High Perfomance®, Q-Sepharose Big Beads® (alle Fa. Pharmacia);
DEAE-Tris-Acryl®, DEAE-Spherodex®, Q-Hyper-D® (alle Fa. Sepracor);
Macroprep DEAE®, Macroprep Q® (alle Fa. BioRad);
DEAE-Toyopearl®, QAE-Toyopearl®, Toyopearl Super-Q® (alle Fa. Tosohaas);
Protein PAK DEAE® (Waters);
Fractogel EMD-TMAE®, Fractogel EMD-DEAE®, Fractogel EMD-DMAE®, Licrospher 1000 TMAE®, Licrospher 1000 DEAE® und Licrospher 4000 DMAE® (alle Fa. MERCK).

Insbesondere werden druckstabile Ionenaustauscher eingesetzt wie z.B. Fractogel TMAE-EMD, Express IonQ, Sonree 30Q. Es hat sich überraschenderweise gezeigt, dass sogar an diesen Materialien das Phänomen der Aktivierung des Faktor VII unterbleibt sobald die Kontaktzeit mit dem Anionenaustauschermaterial bzw. die Verweilzeit auf der Säule kurz gehalten wird z.B. weniger als 5 min. In einer Säule wird dementsprechend vorzugsweise eine Flussrate von mindestens 2,5 cm/Minute, vorzugsweise 3,0 cm/Minute, für die Elution des adsorbierten Faktor VII gewählt.

Die Flussrate entspricht mindestens 0,15 Säulenvolumina pro Minute, vorzugsweise 0,17, am meisten bevorzugt 0,2 Säulenvolumina pro Minute.

Zur Herstellung eines Faktor VII-Präparates kann beispielsweise die Kombination der Anionenaustauscherchromatographie und hydrophoben Interaktionschromatographie eingesetzt werden. Gegebenenfalls kann anschließend noch zur weiteren Reinigung eine Gelfiltration vorgenommen werden. Dementsprechend wird als Chromatographiematerial ein Anionenaustauscher eingesetzt und ein für die hydrophobe Chromatographie geeignetes Material.

Der erfindungsgemäß gereinigte Faktor VII kann nicht nur durch die üblichen Maßnahmen der Dialyse/Diafiltration, Sterilfiltration und Konzentration zu einem pharmazeutischen Faktor VII-Präparat formuliert werden. Ebenso können auch Kombinationspräparate zur Verfügung gestellt werden, welche den erfindungsgemäß gereinigten Faktor VII neben anderen Wirksubstanzen enthalten.

Erfindungsgemäß wird ein Präparat auf Basis von Blutgerinnungsfaktor VII mit einem Anteil von weniger als 5% Faktor VIIa, bezogen auf die Gesamtmenge Faktor VII, mit einer spezifischen amidolytischen Aktivität von mindestens 50 E/mg und einer Stabilität in Abwesenheit von Inhibitoren der Blutgerinnung, das frei ist von Inhibitoren der Blutgerinnung, erhalten.

Erfindungsgemäß ist es erstmals möglich, ein hochgereinigtes Faktor VII-Präparat herzustellen ohne Verwendung der bekannten Inhibitoren der Blutgerinnung, insbesondere ohne Zusatz von Antithrombin III und/oder Heparin, Benzamidin, Sojabohnen-TrypsinInhibitor, Phenylmethylsulfonylfluorid oder EDTA. Es hat sich herausgestellt, dass unter den nachfolgend beschriebenen Bedingungen der Faktor VII während eines chromatographischen Reinigungsverfahrens auch ohne den Schutz vor proteolytischen Enzymen bzw. Kontaktaktivierung nicht aktiviert wird. Somit enthält das hochgereinigte Faktor VII-Präparat keinen der genannten Inhibitoren bzw. weniger als die Nachweisgrenze. Sobald eine spezifische Aktivität von mindestens 50 E/mg erhalten wurde, konnte überraschenderweise gezeigt werden, dass der Faktor VII außerordentlich stabil ist (sogar bezüglich autokatalytischer Aktivierungsprozesse), ohne dass es den Zusatz eines spezifischen FVII-Aktivierungs-Inhibitors bedarf.

Die Stabilität des hochgereinigten Faktor VII kann vor allem - jedoch nicht ausschließlich (vgl. Pedersen et al., 1989 bzgl. autokatalytischer Aktivierung) - auf die Abreicherung der Faktor VII-spezifischen Proteasen, darunter die Faktoren IXa und Xa, zurückgeführt werden. Damit ist gewährleistet, dass ein Präparat, wie ein pharmazeutisches Infusionspräparat, auf Basis des hochgereinigten Faktor VII mit mindestens 50 E/mg Protein, vorzugsweise mindestens 100 E/mg, am meisten bevorzugt mindestens 500 E/mg Protein, in speziellen Fällen sogar mindestens 1000 E/mg bis zur theoretischen Reinheit von etwa 2000 E/mg, auch in Abwesenheit von Inhibitoren der Blutgerinnung über einen längeren Zeitraum in gebrauchsfertigem Zustand verbleiben kann, ohne den Anteil an Faktor VIIa im Präparat über das zulässige Maß zu erhöhen.

Das erfindungsgemäß erhaltene Präparat hat einen Anteil von weniger als 5% Faktor VIIa bezogen auf die Gesamtmenge an Faktor VII, vorzugsweise weniger als 3%, am meisten bevorzugt weniger als die Nachweisgrenze (z.B. im Test nach Seligson et al., Haemostasis 13, 186-191, 1983).

Das erfindungsgemäß erhaltene Präparat ist beispielsweise ein Konzentrat mit pharmazeutischer Qualität, welches zur Herstellung von pharmazeutischen Kombinationspräparaten eingesetzt werden kann. Derartige Kombinationspräparate können weitere Wirksubstanzen, wie Vitamin K-abhängige Proteine, darunter einen oder mehrere der Blutfaktoren II, IX, X, Protein C oder Protein S enthalten. So kann etwa ein Prothrombinkomplex-Präparat enthaltend die Faktoren II, VII, IX und X durch Zumischen des erfindungsgemäßen Faktor VII-Präparates zu einem partiellen Prothrombinkomplex hergestellt werden.

Das erfindungsgemäß erhaltene Faktor VII-Infusionspräparat kann aufgrund der geringen Belastung mit Verunreinigungen in relativ hoch konzentrierter Form zur Verfügung gestellt werden, beispielsweise mit einer Konzentration von 50 bis 5000 E/ml. Damit wird die Verabreichung des Präparates als Bolus-Injektion oder kurzzeitige Infusion wesentlich vereinfacht.

Die Stabilität des Präparates kann in gebrauchsfertigem Zustand getestet werden, ob es die Stabilitätskriterien erfüllt, und zwar durch Inkubation bei Raumtemperatur über einen Zeitraum von mindestens 12 Stunden, vorzugsweise mehr als 30 Stunden. Dabei kann festgestellt werden, dass das erfindungsgemäß erhaltene Präparat immer noch weniger als 5% Faktor VIIa enthält.

Das erfindungsgemäß erhaltene Präparat kann aber auch in einer haltbaren Handelsform zur Verfügung gestellt werden, vorzugsweise als Lyophilisat. Nach Rekonstitution zeigt sich wieder die außerordentliche Stabilität und es kommt auch während der Lyophilisierung/Rekonstitution zu keinerlei negativen Effekten bezüglich der (vorzeitigen) FVII-Aktivierung. Weitere Formen sind tiefgefrorene Präparate oder Flüssigpräparate, welche gegebenenfalls nach Zusatz von Stabilisatoren, wie Trägerproteine und/oder Kohlenhydrate, vorzugsweise bei 4°C über einen längeren Lagerungszeitraum stabil sind.

Der Faktor VII im erfindungsgemäß erhaltene Präparat ist - trotz seiner Stabilitätseigenschaften (auch bezüglich Autoaktivierung) - jedenfalls ein aktivierbarer Faktor VII, der ohne weiteres z.B. *in vivo* aktiviert werden kann, und dann entsprechend dem nativen Faktor VII blutgerinnungsaktiv ist. Vorzugsweise wird ein natives Faktor VII-Protein eingesetzt, beispielsweise humaner plasmatischer Faktor VII, oder humaner rekombinanter Faktor VII. Bei der Rekombination von Nukleinsäuren können auch Faktor VII-Analoge eingesetzt werden, welche jedenfalls gleichermaßen oder in einem erhöhten Ausmaß aktivierbar sind (Sridhara et al., Am. J. Hematology 53, 66-71, 1996).

Insbesondere kann ein Prothrombinkomplex-Präparat erfindungsgemäß zur Verfügung gestellt werden, welches neben dem hochgereinigten und stabilen Faktor VII noch mindestens einen der Blutgerinnungsfaktoren II, IX und X enthält. Diese weiteren Blutgerinnungsfaktoren sind vorzugsweise ebenfalls als Einzelfaktoren gereinigt, bevor das Kombinationspräparat durch entsprechendes Formulieren hergestellt wird. Weiter kann ein Heparingehalt vorgesehen werden, entsprechend einer Empfehlung von Menache et al., Thrombosis Diathes. Haemorrh. 33, 645-647, (1975) zur Bereitung von Faktor IX-hältigen pharmazeutischen Präparaten.

Die Erfindung wird durch die nachfolgenden Beispiele noch weiter beschrieben.

### Beispiel 1 : Reinigung von Faktor VII aus Kryoüberstand durch Anionenaustauscherchromatographie an Fraktogel TMAE-EMD

340 l Kryoüberstand werden an Al(OH)₃ adsorbiert und mit 22,5 g Na₂HPO₄ x 2 H₂O/l (pH 8,5) enthaltend 1 % (V/V) Tween 80 (-pflanzlich) eluiert und mit AT III/Heparin-Komplex versetzt (350 IE Heparin/kg Eluat, 40 IE AT III/kg Eluat). Das Tween-haltige Eluat wird durch Ultrafiltration an einer Membran mit einer Ausschlussgrenze ≤ 30 kD ca. 15-fach konzentriert und gegen 10 Volumina 20 mM Tris/HCl (pH 7,0) (Tris-Puffer) diafiltriert. Nach 0,2 µ-Filtration und Einstellen der Tweenkonzentration auf 15 % (V/V) wird zur Virusinaktivierung 3 Stunden lang bei 40°C inkubiert. Die mit Trispuffer auf das doppelte Volumen verdünnte Lösung (3 1) wird auf eine BPG100/165 Fraktogel TMAE-EMD 650 M-Säule (Fa. MERCK) aufgetragen, mit Trispuffer nachgewaschen und anschließend mit steigenden NaCl-Stufengradienten (50, 100, 150, 200, 250, 1000 mM/l) in Trispuffer gewaschen, eluiert und regeneriert. Die Flussrate beträgt bei der Betthöhe von 16,5 cm mindestens 2,5 bis 3 cm/min.

Das 200 mM-NaCl-Eluat (ca. 5 l) wird nach Zusatz von 43,8 IE Heparin/kg und 5,0 IE ATIII/kg durch Ultrafiltration an einer Membran mit einer Ausschlussgrenze ≤ 30 kD ca. 60-fach auf eine Proteinkonzentration von 5 mg/ml konzentriert. Nach Diafiltration gegen eine Lösung von 4,8 mM Na₃-Citrat x 2 H₂O und 61,6 mM NaCl/l wird der pH auf einen Wert von 8,0 +/- 0,5 eingestellt und die Lösung eingefroren und lyophilisiert. Das Lyophilisat wird bis zu einer Restfeuchte von 7 - 8% befeuchtet und für die Virusinaktivierung 10 Stunden bei 60°C und 1 Stunde bei 80°C erhitzt.

**Tabelle 1**

| **Ergebnis** | | | |
|---|---|---|---|
| Fraktion | Spezif. Akt.* [E FVII/mg Prot.] | FVII-Aktivierung** [E FVIIa/E FVII] | Reinigungsfaktor |
| Plasma | 0,02 | - | 1 |
| Al(OH)3-Eluat n. Tween | 5 | 0,05 | 200 |
| TMAE-Eluat | 100 | 0,25 | 5.000 |
| hitzebehandeltes Lyophilisat | 100 | 0,35 | 5.000 |

| | | | |
|---|---|---|---|
| * : maximale theoretische spezifische Aktivität: 2000 E FVII/mg Protein | | | |
| ** : vollständige Aktivierung bei einem FVIIa/FVII-Verhältnis von 15-20 | | | |

### Beispiel 2 : Reinigung von Faktor VII aus Kryoüberstand durch Anionenaustauscherchromatographie an Fraktogel TMAE-EMD und nachfolgender hydrophober Chromatographie an Phenyl-Sepharose

Bis zur Herstellung des hitzebehandelten Präparates (Bulkpulver) wird wie in Beispiel 1 verfahren. Das Bulkpulver wird im ursprünglichen Volumen mit Milli Q-Wasser (Fa. Millipore) gelöst (Proteinkonzentration ca. 5 mg/ml), von 60 auf 2000 mM NaCl/l aufgesalzen und auf eine XK50/96 Phenyl-Sepharose HP-Säule (Fa. Pharmacia) aufgetragen, die in 20 mM Tris/HCl (pH 7,4; 2000 mM NaCl/l) equilibriert worden war. Nach Auftrag von 75 ml FVII-Bulkpulverlösung bei einer Flußrate von 10 ml/min wird mit ca. 10 SV Equilibrierungspuffer nachgewaschen und anschließend mit den folgenden NaCl-Stufen gewaschen, eluiert bzw. regeneriert:
1200 mM NaCl/l
850 mM NaCl/l
500 mM NaCl/l
0 mM NaCl/l, jeweils in 20 mM Tris/HCl (pH 7,4)

Ca. 2,8 l des 850 mM NaCl-Eluats werden anschließend durch Ultrafiltration an einer Membran mit einer Ausschlußgrenze ≤ 30 kD 5-fach konzentriert und gegen 20 mM Ammoniumhydrogencarbonat diafiltriert und unter Sublimation der Salze lyophilisiert. Das salzfreie Lyophilisat wird in 1/50 des ursprünglichen Volumens in 0,4% Na,-Citrat x 2 H₂O 0,8 % NaCl (pH 7,0) aufgenommen und an einer im gleichen Puffer equilibrierten XK26/100 Superose 12-Säule (Fa. Pharmacia) unter geringer Aufreinigung bei einer Flußrate von 2,5 ml/min umgepuffert.

**Tabelle 2**

| **Ergebnis** | | | | |
|---|---|---|---|---|
| Fraktion | Spezif. Akt. [E FVII/mg Prot.] | FVII-Aktivierung [E FVIIa/E FVII] | F VII-Ausbeute [%] | Reinigungsfaktor |
| Plasma | 0,02 | - | - | 1 |
| Al(OH)3-Eluat n.Tween | 5 | 0,05 | 100 | 200 |
| TMAE-Eluat | 100 | 0,25 | 80 | 5.000 |
| hitzebehandeltes Lyophilisat | 100 | 0,35 | 61 | 5.000 |
| Phenyl-Seph.-Eluat | 500 | 0,4 | 43 | 25.000 |
| Superose 12-Eluat | 1000 | 0,5 | 35 | 50.000 |

### Beispiel 3 : Einfluß der Flußrate auf die Aktivierung von Faktor VII an Fraktogel TMAE-EMD

An einer XK26/16,5 Fraktogel TMAE-EMD 650 M-Säule (Fa. MERCK) wurde die Aufreinigung von FVII aus Al(OH)3-Eluat nach Virusinaktivierung mit 15 % Tween (Auftrag 36 mg Protein/ml Gel) bei verschiedenen Flußraten bei 22°C getestet. Die Bedingungen der Chromatographie entsprechen Beispiel 1.

**Tabelle 3**

| **Ergebnis** | |
|---|---|
| **Flußrate [cm/min]** | **Aktivierung [E F7a** / **E F7]** |
| 0,94 | 7,6 |
| 1,88 | 2,65 |
| 2,35 | 0,45 |
| 2,83 | 0,24 |

Es zeigt sich, daß bei zunehmender Flußgeschwindigkeit der Gehalt an aktiviertem Faktor VII abnimmt. Die Aktivierungsrate bleibt dann ab einem Wert von über etwa 2,5 bis 3 cm/s ungefähr konstant.

### Beispiel 4 : Test zur Bestimmung der Stabilität der FVII-Präparation

### 4.1. Inkubationsbedingungen

100-300 µl-Aliquots der FVII-haltigen Eluate der TMAE- bzw. Phenyl-Sepharose-Chromatographie, hergestellt gemäß Beispiel 1 und 2, wurden für 38 Stunden bei 22°C inkubiert und nach dieser Zeit die amidolytische FVII-Aktivität (Immunochrom FVII:C, Fa. IMMUNO AG), die FVIIa-Gerinnung (Staclot VIIa-Rtf, Fa. Diagnostica Stago) und die Proteinkonzentration (Bradford) im Vergleich zu einem sofort bei -20°C eingefrorenem Aliquot getestet.

### 4.2. Aktivitätstests

### 4.2.1. IMMUNOCHROM FVII:C

Die Faktor VII-Aktivität wurde unter Bedingungen einer vollständigen Aktivierung von FVII durch Thromboplastin und Ca²⁺ und der dann folgenden Aktivierung von ebenfalls zugesetztem FX mit einem chromogenen FXa-Substrat kinetisch gemessen. Es wurde entsprechend den Empfehlungen des Herstellers verfahren.

### 4.2.2. STACLOT VIIa-rTF

Mit rekombinantem löslichen Tissue factor läßt sich in Gegenwart von Phospholipid und FVII-Mangelplasma allein die durch FVIIa ausgelöste Gerinnung mit Hilfe eines Coagulometers messen. Es wurde nach der Vorschrift des Herstellers Diagnostica Stago verfahren.

### 4.3. Ergebnis

| Fraktion | Protein. [mg/ml] | E FVII chrom/mg Spez. Akt. | E FVIIa Aktivier. t=0 | E FVIIa Aktivier. t=38 |
|---|---|---|---|---|
| TMAE, 200 mM | 0,04-0,08 | 150-200 | 0,1 - 0,3 | 0,3 - 0,8 |
| Phenyl-Seph., 750 mM | 0,02-0,04 | 500-1000 | 0,1 - 0,2 | 0,1- 0,3 |

Es zeigt sich, daß bei den erfindungsgemäß hergestellten Präparaten selbst bei 38-stündiger Lagerung bei 22°C keine nennenswerte Aktivierung von FVII auftritt. Dies ist um so überraschender, als bei bislang bekannten Faktor VII-Präparaten immer eine erhebliche Aktivierung (u.a. auch durch Autokatalyse) eingetreten ist, die nur durch den Zusatz von spezifischen Inhibitoren verhindert bzw. hintangestellt werden konnte.

## Patentansprüche

1. Verfahren zur Reinigung von Faktor VII aus einem biologischen Material und Herstellung eines Faktors VII-Präparates durch Adsorption des Faktor VII an einen Anionen-Austauscher in einer Säule, fraktionierte Elution des Faktor VII mit einer spezifischen amidolytischen Aktivität von mindestens 50 E/mg, wobei die Elution mit einem Puffer ohne Zusatz von Inhibitoren der Blutgerinnung vorgenommen wird und die Flussrate der Elution mindestens 0,15 Säulenvolumina pro Minute beträgt, und Gewinnen des Faktor VII aus dem Eluat, so dass das Faktor VII-Präparat einen Anteil von weniger als 5% Faktor VIIa, bezogen auf die Gesamtmenge Faktor VII, aufweist.

2. Verfahren nach Anspruch 1, **dadurch gekennzeichnet, dass** der Faktor VII aus Blut, Plasma, einer Plasmafraktion, einer Zellkultur oder einer Zellkulturfraktion gereinigt wird.

3. Verfahren nach Anspruch 1 oder 2, **dadurch gekennzeichnet, dass** der Faktor VII aus der eluierten Fraktion gewonnen wird, die den Faktor VII mit einer spezifischen amidolytischen Aktivität von mindestens 100 E/mg enthält.

## Claims

1. A method for purifying factor VII from a biological material and producing a factor VII preparation by adsorption of the factor VII on an anion exchanger in a column, fractionated elution of the factor VII with a specific amidolytic activity of at least 50 U/mg, wherein the elution is carried out with a buffer with no addition of blood coagulation inhibitors, and the elution flow rate is at least 0.15 column volumes per minute, and recovery of factor VII from the eluate, so that the factor VII preparation comprises a portion of less than 5% of factor VIIa, based on the total amount of factor VII.

2. A method according to claim 1, **characterized in that** the factor VII is purified from blood, plasma, a plasma fraction, a cell culture or a cell culture fraction.

3. A method according to claim 1 or claim 2, **characterized in that** the factor VII is extracted from the eluted fraction which contains the factor VII with a specific amidolytic activity of at least 100 U/mg.

## Revendications

1. Procédé pour purifier le facteur VII à partir d'un matériau biologique et production d'une préparation à base d'un facteur VII par adsorption du facteur VII sur un échangeur d'anions dans une colonne, élution fractionnée du facteur VII avec une activité amidolytique spécifique d'au moins 50 U/mg, l'élution étant entreprise avec un tampon sans ajout d'inhibiteurs de la coagulation sanguine et le débit de l'élution s'élevant au moins à 0,15 volume de colonne par minute, et la récupération du facteur VII à partir de l'éluat, de sorte que la préparation à base de facteur VII présente une fraction de moins de 5 % de facteur VIIa sur la base de la quantité totale de facteur VII.

2. Procédé selon la revendication 1, **caractérisé en ce que** le facteur VII est purifié à partir de sang, de plasma, d'une fraction plasmatique, d'une culture cellulaire ou d'une fraction de culture cellulaire.

3. Procédé selon la revendication 1 ou 2, **caractérisé en ce que** le facteur VII est recueilli à partir de la fraction éluée, qui contient le facteur VII avec une activité amidolytique spécifique d'au moins 100 U/mg.
